# EUROPEAN PATENT APPLICATION

(11) **EP 2 320 235 A1**
(43) Date of publication of application: **11.05.2011**
(21) Application number: 09175249.3
(22) Date of filing: 06.11.2009
(51) Int. Cl.: G01N 33/574

(54) **Marker for prostate cancer diagnosis**

(71) Applicant: IMG Institut für medizinische Genomforschung Planungsgesellschaft M.B.H., 1010 Vienna (AT)
(72) Inventor: Klocker, Helmut, 6401, Inzing (AT); Schäfer, Georg, 6020, Innsbruck (AT); Chui Pressinotti, Nicole, 70197, Stuttgart (DE); Sültmann, Holger, 67117, Limburgerhof (DE); Van Duc, Luu, 8032, Zurich (CH)
(74) Representative: Sonn & Partner Patentanwälte

(57) **Abstract**

The invention relates to the use of PDIA3 as a marker for prostate cancer diagnosis, especially for distinguishing significant high-grade prostate carcinoma from insignificant low-grade prostate carcinoma in a sample of a prostate cancer patient.

SEQUENCE LISTING

<110>  IMG INSTITUT FÜR MEDIZINISCHE GENOMFORSCHUNG
       PLANUNGSGESELLSCHAFT M.B.H.

<120>  Marker for Prostate Cancer Diagnosis

<130>  R 53591

<160>  4

<170>  PatentIn version 3.4

<210>  1
<211>  19
<212>  RNA
<213>  artificial

<220>
<223>  target sequence

<400>  1
ggaauagucc cauuagcaa                                                    19


<210>  2
<211>  19
<212>  RNA
<213>  artificial

<220>
<223>  target sequence

<400>  2
gggcaaggac uuacuuauu                                                    19


<210>  3
<211>  19
<212>  RNA
<213>  artificial

<220>
<223>  target sequence

<400>  3
agacccaaau aucgucaua                                                    19


<210>  4
<211>  19
<212>  RNA
<213>  artificial

<220>
<223>  target sequence

<400>  4
gaggaguucu cgcgugaug                                                    19

## Description

The present invention relates to a novel marker in diagnosing prostate cancer (PC).

Prostate cancer (PC) is the most frequent cancer diagnosed in men (20.3% of the total), followed by lung (17.2%) and colorectal cancer (12.8%). Measuring prostate specific antigen (PSA) has been a matter of routine to detect prostate cancer, but is insufficient to distinguish between different tumour grades. The widespread use of PSA for the detection of PC has resulted in an increasing number of men diagnosed with organ-confined, low Gleason-score PC, which are potentially curable. However, the high sensitivity of the PSA test is accompanied by a low specificity, causing many patients suffering from unnecessary biopsy taking. Men with normal prostate pathology generally have a PSA level in blood below 4ng/ml. PSA levels between 4ng/ml and 10ng/ml ("grey zone") have a 25% chance of having prostate cancer. However, some men with prostate cancer may have normal levels of PSA and elevated levels of PSA may also be due to other factors, such as age, infarction etc.. As a consequence, in 75% of the cases, men with an abnormal DRE and a PSA in this grey zone have a negative, or a seemingly unnecessary biopsy.

The Gleason Grading System is commonly used for histological-based grading of prostate cancer tissue. Since prostate tumours are often multifocal, the Gleason Score (GS) is the sum of the two most prevalent tumour patterns, which are graded 1 (CA1) as the most differentiated to 5 (CA5) as the least differentiated pattern of cancerous gland. Other methods for subclassification have been described in recent reports. These indicate that translocations fusing the strong androgen-responsive gene, TMPRSS2, with ERG or other oncogenic ETS factors may facilitate prostate cancer development. It has been proposed that the presence or absence of this genetic rearrangement may be used, much like the Gleason grading system, as a diagnostic tool to extract prognostically relevant sub-classifications of this cancer.

The discrimination between different tumour grades is important with respect to treatment decisions: Currently, many men who are diagnosed with GS 6 prostate cancer are often "over"-treated and risk suffering from urinary and sexual dysfunction - frequent side effects of treatment. Therefore, it is also important to develop a sensitive and specific diagnostic tool to distinguish between different tumour grades. To address this problem, many groups have recently started to profile expression levels of prostate cancer to identify deregulated genes during disease progression. However, although many of these have addressed the question of molecular differences between normal, tumour, benign prostatic hypertrophy (BPH), and the putative precursor lesion prostatic intraepithelial neoplasia (PIN), little is still known about molecular changes between low- and high-risk tumours. One hallmark of highly aggressive tumours is their resistance towards apoptosis. Defects in the apoptotic signaling machinery are a key factor for the survival of malignant cells, and also contribute to drug resistance of tumour cells. Many tumour cells of higher stages have also acquired the ability for higher migration and invasion potential. These transformation steps involve changes in intracellular signaling pathways that regulate, amongst other events, actin-based motility. In addition, fast proliferating cells require high supply of energy, therefore deregulation of genes participating in metabolic processes has been described in aggressive tumour cells.

It is therefore an object of the present invention to provide means for diagnosing PC in a sample taken from an individual. These means should preferably be reliable, suitable for routine testing and have high sensitivity and high specificity to enable testing of a large number of samples and eventually be carried out by automatic devices. Preferably, such means should be suitable for distinguishing between low- and high-risk tumours.

Therefore, the present invention provides the use of Protein disulfide-isomerase A3 (PDIA3) as a marker for diagnosis of prostate cancer.

Preferably, the present invention provides the use of PDIA3 as a marker for distinguishing significant high-risk prostate carcinoma from insignificant low-risk prostate carcinoma in a tissue sample, especially a prostate biopsy tissue, sample, preferably by the following steps:
- identifying a Gleason 3 region in the tissue sample;
- determining the expression of PDIA3 in the Gleason 3 region identified; and
- identifying the tissue sample as being derived from a significant high-risk prostate carcinoma if the expression level of PDIA3 determined are increased compared to the expression level of PDIA3 in a Gleason 3 region of an insignificant low-risk prostate carcinoma.

Preferably, the present invention can also be based on a specific combination of markers for diagnosing PC or for confirming Gleason Pattern in a sample, especially a prostate biopsy. Accordingly, PDIA3 can be combined with other markers, such as PSA. In confirming Gleason Pattern 3, the combination of PDIA3, NPM1 and VDAC1 protein or the amount of mRNA expression of PDIA3, NPM1 and VDAC1 may be employed in the diagnosis, staging, progression, monitoring and/or prognosing PC in a sample of a tumour patient or an individual being at risk or being suspected of having a tumour.

The PDIA3 gene encodes a protein of the endoplasmic reticulum that interacts with lectin chaperones calreticulin and calnexin to modulate folding of newly synthesized glycoproteins. The protein was once thought to be a phospholipase; however, it has been demonstrated that the protein actually has protein disulfide isomerase activity. It is thought that complexes of lectins and this protein mediate protein folding by promoting formation of disulfide bonds in their glycoprotein substrates.

PDIA3 is in the EC class EC 5.3.4.1, it has also been termed ER60, ERP57, ERP60, ERp57, ERp60, ERp61, GRP57, GRP58, HsT17083, OTTHUMP00000041709, P58, PI-PLC orp58. The amino acid/nucleic acid sequences are listed in the following databases under the numbers given: HGNC: 4606; Entrez Gene: 2923; UniProtKB: P30101; Ensembl: ENSG00000167004; NCBI: NM_005313.

The role of PDIA3 as a single marker for prostate cancer in general or as a marker for specific prostate cancer stages was previously regarded differently in the prior art: Low expression levels of PDIA3 were understood as indicating higher Gleason scores: Tomlins et al. (Neoplasia 10 (2007), 177-188) identified PDIA3 as one of the top 20 underexpressed genes (GS6 vs. GS8-10), downregulation is confirmed by GNF SymAtlas Cancer data; Earlier, Dhanasekaran et al. (Nature 412 (2001), 822-826) reported a down-regulation of PDIA3 in metastatic, late stage prostate cancer. In contrast thereto, the present invention uses an increased PDIA3 level as part of the marker combination according to the present invention for PC indication. It is only the present invention, which provides PDIA3 in a usable manner for prostate cancer diagnosis.

According to the present invention, PDIA3 has been proven as reliable markers for distinguishing prostate tumours and benign prostatic tissue including BPH, especially for distinguishing between high- and low-risk tumours in a Gleason Pattern 3 sample. In addition it could be shown that the combination of PDIA3, NPM1 and VDAC1 provides a good and reliable marker set for prostate cancer diagnostics according to the present invention. In addition, the marker combination also enabled a statistically significant differentiation between Gleason Pattern 3 and 4 and can therefore be used for verifying Gleason Pattern 3 in the method according to the present invention. This provides effective means for distinguishing significant high-grade prostate carcinoma from insignificant low-grade prostate carcinoma.

In fact, the role of PDIA3 as a powerful marker for diagnosing prostate cancer, especially for diagnosing the important borderline between a significant high-grade (=high risk) prostate carcinoma and an insignificant low-grade (=low risk) prostate carcinoma, according to the present invention has also to be viewed as a surprising explanation for unclear and conflicting results in the prior art. For example, it is confirmed by the present invention that in highly dedifferentiated prostate cancer tissue (Gleason 5 (CA5)) PDIA3 expression is low; however, not at the critical borderline to which the present invention attends in a preferred embodiment. According to the present invention, the expression level of PDIA3 in Gleason 3 areas (CA3) can vary depending whether higher grade areas (CA4 or CA5) are present in the tumour or not: If higher grade areas (CA4 or CA5) are present in the tumour, PDIA3 expression in CA3 is significantly higher than in tumours with no CA4 or CA5 areas. Accordingly, if increased expression of PDIA3 is found in CA3 in a tumour biopsy, the probability that higher Gleason Patterns are present (and that therefore the tumour in fact is GS > 6 is significantly increased. This shows that with this specific differentiated analysis, PDIA3 is a superior marker in prostate cancer. If this analysis is performed PDIA3 can be used as a highly reliable marker, not only for diagnosing of PC (and to distinguish PC from benign prostate tissue and PIN), but mainly to differentiate between two important groups of prostate patients: those who need aggressive treatment regimes and those for whom - for the time being - careful observation of the further development of the tumour is sufficient.

Surprisingly, PDIA3 turned out to be a highly reliable marker for distinguishing between significant high-grade prostate carcinoma (which requires aggressive treatment regimes, including prostatectomy) and an insignificant low-grade prostate carcinoma (which usually requires "watchful waiting" only).

The present invention therefore also provides a method for distinguishing significant high-grade prostate carcinoma from insignificant low-grade prostate carcinoma in a patient comprising the following steps:
- providing a tissue sample, especially a prostate biopsy sample, of said patient;
- identifying a Gleason 3 region in the tissue sample;
- determining the expression of PDIA3 in the Gleason 3 region identified; and
- identifying the tissue sample as being derived from a significant high-grade prostate carcinoma if the expression levels of PDIA3 determined is increased compared to the expression levels of PDIA3 in a Gleason 3 region of an insignificant low-grade prostate carcinoma.

The present invention preferably relates to the use of expression levels of PDIA3 in a Gleason 3 region of a tissue sample of a prostate carcinoma for distinguishing significant high-grade prostate carcinoma from insignificant low-grade prostate carcinoma wherein increased expression levels of PDIA3 identify a significant high-grade prostate carcinoma and wherein a non-increased or decreased expression levels of PDIA3 identify an insignificant low-grade prostate carcinoma.

The present invention also provides a method for diagnosing prostate cancer in a patient comprising the follows step:
- providing a tissue sample or a body fluid sample of said patient;
- determining the amount of PDIA3 or the amount of expression of PDIA3 in the sample; and
- comparing the amount of PDIA3 or the amount of expression of PDIA3 in the sample with a sample of known prostate cancer status.

The method for determining the expression level of PDIA3 is not critical as long as it allows the in vitro determination of PDIA3 protein or PDIA3 mRNA in the sample. In prostate tissue sample biopsies it is specifically preferred that the method allows determination of CA3-specific (expression) levels of PDIA3. Preferably, the expression or amount of PDIA3 in tissue specimen, especially in the Gleason 3 region identified, is determined by immunohistochemical (IHC) methods, by immunofluorescence (IF) methods, by RNA in-situ hybridisation, by reverse transcriptase polymerase chain reaction (RT-PCR), especially quantitative real time RT-PCR (qRT-PCR), or by a combination of these methods. Such samples could preferably be taken by manual or microscopical microdissection. Other methods for determining the level of (expression of) PDIA3 include MALDI-MS (e.g. WO 2009/004576 A (including surface enhanced laser desorbtion/ionization mass spectrometry (SELDI-MS), especially surface-enhanced affinity capture (SEAC), surface-enhanced need disorption (SEND) or surface-enhanced photo label attachment and release (SEPAR) for the determination of proteins in samples for diagnosing and staging of prostate cancer)), antibody testing (e.g. WO 2008/031839 (including immunoprecipitation, Western blotting, Enzyme-linked immuno sorbent assay (ELISA), Enzyme-linked immuno sorbent assay (RIA), dissociation-enhanced lanthanide fluoro immuno assay (DELFIA), scintillation proximity assay (SPA) for diagnosing, staging or monitoring prostate cancer samples under investigation of specific marker proteins), and quantitative nucleic acid testing (e.g. in WO 2006/066965 A, especially PCR, LCR and RT-PCR of samples for prostate cancer marker mRNA detection and quantification).

Usually, the amount of PDIA3 protein or the amount of PDIA3 mRNA in the sample is compared with a reference value for this amount with a known prostate cancer status, i.e. a value which is known to be a healthy value (i.e. a value not affected by prostate cancer) or which is known to be a diseased status with respect to prostate cancer, preferably of a given stage of prostate cancer.

Such reference, standard or control samples are all useable in principle for comparison with the sample of unknown prostate cancer status diagnosed according to the present invention. Such reference, standard or control samples can be taken e.g. from a human male subject with negative diagnosis prostate cancer or undetectable ongoing prostate cancer development. If such a control sample, standard sample or reference sample is said to be comparable to the sample that is taken from a human male subject being suspected to be afflicted by ongoing cancer development according to the present invention, this means that both samples, except for their expression profile have been derived and treated equally. Thus, the sample in both cases may e.g. be a tissue or blood derived sample which has been further treated in a way to allow for detection of the diagnostic marker molecules as mentioned above.

Although prostate tissue samples from the patient are preferred samples, the present marker is also useable in other suitable samples of tissue or body fluids. For example, the sample may be a blood sample, or a sample derived from blood, such as e.g. a serum sample or a plasma sample or a fraction of a blood, serum or plasma sample. Samples, which do not need prostate biopsies or prostatectomy performed on the patient for providing the samples are specifically preferred for early staging and diagnosis of prostate cancer.

Blood plasma is the yellow liquid component of blood, in which the blood cells in whole blood would normally be suspended. It makes up about 55% of the total blood volume. It is mostly water (90% by volume) and contains dissolved proteins, glucose, clotting factors, mineral ions, hormones and carbon dioxide. Blood plasma is prepared by spinning a tube of fresh blood in a centrifuge until the blood cells fall to the bottom of the tube. The blood plasma is then poured or drawn off. Blood plasma has a density of approximately 1.025 kg/l. Blood serum is blood plasma without fibrinogen or the other clotting factors (i.e., whole blood minus both the cells and the clotting factors).

The diagnostic methods according to the present invention may also be carried out in saliva, bladder washing, semen or urine samples, especially urine samples after prostate massage.

Although the diagnostic fine tuning for established clinical practice will work with nomograms for defining the differences in marker expression according to the present invention, it is also preferred to establish the diagnostic system with the comparison of the sample to be analysed according to the present invention and a sample of the same source of a known status. It is therefore preferred to use the amounts present of PDIA3 (or the amount of respective PDIA3 mRNA present) in a healthy (prostate) tissue sample and compare these to the (prostate) tissue sample under investigation. If the amount of PDIA3 (or the mRNA) is significantly increased, diagnosis of prostate cancer is indicated, especially if taken from a Gleason Pattern 3. The same is preferred for e.g. blood, plasma, serum, urine, semen or saliva samples: also here suitable comparison values are derived from samples blood, plasma, serum, urine, semen or saliva, respectively, from a patient with has a healthy prostate tumour status, preferable samples taken (earlier) from the same patient. On the other hand, the comparison may also be taken to establish known amounts of PDIA3 (or the mRNA) in the sample taken. As usual in human medical diagnosis, absolute limiting value can be defined for each of the samples to determine difference between healthy and prostate tumour and between different stages of the tumour. These absolute values have to be defined and carefully confirmed depending on the very method applied for determining PDIA3. For the examples according to the present invention, a reliable test system has been established, i.a. based on a scoring system already established for other tumour markers (e.g. the "Remmele score" for mammary carcinoma).

The determination of the expression level of PDIA3 in the cancer area (Gleason 3) of a prostate biopsy identifies this biopsy either being representative for a significant (high-grade) prostate carcinoma in case of an elevated expression level of PDIA3 or representative for an insignificant (low grade) prostate carcinoma in case of not elevated expression levels.

The level of expression or the amount of protein present of PDIA3 according to the present invention is measured and is compared with the level of expression of PDIA3 from other cells or samples. The comparison may be effected in an actual experiment; or intellectually (by comparison with known reference values); or virtually (e.g. automatically in silico). When the expression level (also referred to as expression pattern or expression signature (expression profile)) is measurably different, there is according to the invention a meaningful (i.e. statistically significant) difference in the level of expression. Preferably the difference in protein amount or in expressed mRNA for PDIA3 is at least 5 %, 10% or 20%, more preferred at least 50% or may even be as high as 75% or 100%. More preferred this difference in the level of expression or protein amount is at least 200%, i.e. two fold, at least 500%, i.e. five fold, or at least 1000%, i.e. 10 fold. The expression level for each of the markers according to the present invention expressed higher in a disease sample than in a healthy, normal sample is at least 5 %, 10% or 20%, more preferred at least 50% or may even be 75% or 100%, i.e. 2-fold higher, preferably at least 10-fold, more preferably at least 50-fold, and most preferably at least 100-fold higher in the disease sample. Whether a PDIA3 level is increased in a given detection method can preferably be established by analysis of a multitude of GS6 tumours with the given detection method. This can then form a suitable level from which the "increased" status can be determined; e.g. by the above % difference or - fold change.

An increased expression of PDIA3 in a low aggressive tumour is indicative for a highly aggressive tumour (CA4 or CA5 portion) resulting in a higher GS classification. In biopsy, a randomly hit tumour area is contained. Since the prostate carcinoma is often multifocal and heterogeneous. The biopsy area can therefore represent the tumour as a whole, but not necessarily. Diverse studies show that Gleason 3 patterns in a biopsy only identify to an extent to 50 to 60 % of the cases a GS6 tumour, the other cases containing higher Gleason Patterns and being > GS6 tumours. The present invention provides an improved solution to this problem by providing PDIA3 as a marker for tumour diagnosis.

Preferred embodiments of the present invention rely on the determination of PDIA3 expression levels in Gleason 3 regions of tumour biopsies of prostate cancers. Although the Gleason typing and the Gleason score is well available for the person skilled in the art, a short summary thereof is included herein:
A Gleason score is given to prostate cancer based upon its microscopic appearance. Cancers with a higher Gleason score are more aggressive and have a worse prognosis.
Most often, a urologist will remove a cylindrical sample (biopsy) of prostate tissue through the rectum, using hollow needles, and prepare microscope slides. After a prostate is removed in surgery, a pathologist will slice the prostate for a final examination.

The pathologist assigns Gleason grades (also known as Gleason Patterns) to the tumour. The two predominating grades are added together to get a Gleason score (also known as the Gleason sum). For example, if the predominant Gleason Pattern was grade 3, and the next most prevalent pattern was grade 4, the Gleason score would be 3 + 4 = 7. If there is only one dominating Gleason Pattern then the Gleason score is two times this Gleason Pattern, e.g. Gleason Score = 2 x 3 = 6 when Gleason Pattern 3 is the single predominating Gleason Pattern of a tumour.

The Gleason grade ranges from 1 to 5, with 5 having the worst prognosis. The Gleason score ranges from 2 to 10, with 10 having the worst prognosis.

Gleason scores are associated with the following features:
Grade 1 - The cancerous prostate closely resembles normal prostate tissue. The glands are small, well-formed, and closely packed
Grade 2 - The tissue still has well-formed glands, but they are larger and have more tissue between them.
Grade 3 - The tissue still has recognizable glands, but the cells are darker. At high magnification, some of these cells have left the glands and are beginning to invade the surrounding tissue.
Grade 4 - The tissue has few recognizable glands. Many cells are invading the surrounding tissue
Grade 5 - The tissue does not have recognizable glands. There are often just sheets of cells throughout the surrounding tissue.

A pathologist examines the biopsy specimen and attempts to give a score to the two patterns. First called the primary grade, represents the majority of tumour (has to be greater than 50% of the total pattern seen). Second - a secondary grade - relates to the minority of the tumour (has to be less than 50%, but at least 5%, of the pattern of the total cancer observed). These scores are then added to obtain the final Gleason score. For the present invention, biopsy samples of prostate patients are therefore the preferred tissue sample wherein PDIA3 expression levels are determined.

The core of the present invention is to use PDIA3 as a marker in prostate cancer diagnostics. This invention can further be combined with already existing and established markers for prostate cancer diagnostics, such as PSA (and/or PSA subtypes), PSM (prostate specific membrane antigen), hK2 (human glandular kallikrein 2), AMACR (alpha-methylacyl-CoA racemase), hepsin or PCA3 (prostate cancer antigen 3), among many others. Further markers suitable for co-assessment with PDIA3 are e.g. MAP3K5, ANXA5, VDAC1, NGFRAP1, TEGT, NPM1, VCP, TRAF4, HMGB1, and ROCK1, especially PDIA3, NPM1 and VDAC1 as a preferred marker combination, especially for distinguishing between GP3 and GP4. The methods according to the present invention can further be combined with the markers disclosed in the prior art, preferably those disclosed in Lopergolo et al. (Cancer 115 (2009), 3058-3067), Karakiewicz et al. (Nat. Clin. Pract. Urol. 5 (2008), 82-92) and/or Khan et al. (BJU Int. 92 (2003), 7-11). In such further combinations, the use of PDIA3 as a marker according to the present invention enables a further improvement of those established methods, especially all sorts of immunoassays.

Another important aspect of the present invention is a kit for detecting the amount of PDIA3 in a sample, comprising detection agents for PDIA3.

Preferred detection agents for PDIA3 are antibodies being specific for PDIA3. Such antibodies are well available in the art; commercial anti-PDIA3-antibodies can be purchased also in high purity levels to establish suitable diagnostic kits. In fact, also detection kits for the three protein markers are commercially available. The antibodies to be used according to the present invention can be monoclonal antibodies as well as polyclonal antibodies, i.e. antibodies contained in a polyclonal antiserum, as well as any antibody fragment wherein the binding specificity for PDIA3 is present. Suitable fragments of antibodies include F(ab')2, Fab and Fv fragments. Suitable derivatives of antibodies include scFvs, chimeric and humanized antibodies. Also diagnostic monoclonal antibodies from mice can be applied according to the present invention. These are easy and cost effective to be produced in cell cultures and can easily be standardised for routine testing.

In general, the kit according to the present invention includes binding agents that specifically bind to PDIA3 protein. These binding agents are preferably linkable or already operably linked to a detectable label. An increased binding of the binding agent specifically bound to PDIA3 protein in the sample of the patient compared to the binding agent bound to PDIA3 protein in a sample of known prostate cancer status, e.g. a sample from a healthy individual indicates the prostate cancer status of the patient. In this embodiment of the invention, a medical imaging device or system detects the binding agent specifically bound to PDIA3. Preferably, the binding agent is immobilised on a solid surface in order to allow simple confirmation of the binding event. Examples for such solid surfaces include microtiter plates, microarrays, ELISA plates, etc.. Exemplary binding agents include natural ligands, synthetic small molecules, chemicals, nucleic acids, peptides, proteins, antibodies and fragments thereof. As already stated above, antibodies against PDIA3 and fragments thereof specifically binding to PDIA3 are preferred binding agents according to the present invention.

Exemplary detectable labels include fluorophores, chemical dyes, radioactive compounds, chemiluminescent compounds, magnetic compounds, paramagnetic compounds, enzymes that yield a coloured product, enzymes that yield a chemiluminescent product and enzymes that yield a magnetic product. These labels may be linked to a secondary binding agent, e.g. an antibody specifically recognising the PDIA3 binding agent. Various methods for detection of PDIA3 are disclosed e.g. in US 6,670,166 B1, US 7,413,851 B2 and US 7,276,588 B2 or easily adaptable to PDIA3.

According to a preferred embodiment, the PDIA3 binding agents are detectable by a different label than other markers which are tested, preferably detectable in one and the same assay, e.g. by different fluorophores, different dyes, different colour developing agent systems, etc..

According to a preferred embodiment for determining the amount of PDIA3 in a prostate tissue biopsy sample, PDIA3 and each additional marker can be detected in consecutive tissue slices (preferably about 4-40 µm thickness). Alternatively, a multiple staining can be performed via immunofluorescence.

For IHC staining, a double staining with P63, a marker for basal cells (Weinstein et al., Mod. Pathol. 15 (2002): 1302-1308) is preferred which allows a discrimination between tumour cells and benign areas.

According to an alternative embodiment of the present invention, a kit for detecting the expression level of PDIA3 in a sample, is provided, comprising detection agents for PDIA3 mRNA.

The kit can comprise suitable primer pairs specific for PDIA3 mRNA and/or reagents enabling a quantitative detection of PDIA3 mRNA in a given sample. The kit may also contain instructions for determining prostate cancer diagnosis and prognosis based on the detection of the particular marker combination according to the present invention. The present kits are specifically useful for diagnosing a tissue sample or a sample of a body fluid for prostate cancer, especially for distinguishing significant high-grade prostate carcinoma from insignificant low-grade prostate carcinoma.

The kit may alternatively or in combination also comprise specific oligonucleotides hybridising to PDIA3 mRNA. Such specific oligonucleotides may act as probes for the PDIA3 mRNA in the sample and can be used to quantitate the specific mRNA in the sample. Preferably, the oligonucleotide probes can be immobilised, e.g. on a suitable microarray. Also established microarray systems, as e.g. the Affymetrix chip, can be used for the diagnosis according to the present system.

Amplification assays, such as PCR, especially qRT-PCR, may be adapted for real time detection of multiple amplification products (i.e., multiplex real time amplification assays). Suitable primer pairs can be made based on the known sequences of the PDIA3 mRNA available e.g. from the sources mentioned above (NCBI, HGNC, etc.).

Such a kit may further include additional components such as additional oligonucleotides or primers and/or one or more of the following: buffers, reagents to be used in the assay (e.g., wash reagents, polymerases or internal control nucleic acid or cells or else) and reagents capable of detecting the presence of bound nucleic acid probe or primers. Examples of detection reagents include, but are not limited to radiolabelled probes, enzymatic labeled probes (horse radish peroxidase, alkaline phosphatase), and affinity labeled probes (biotin, avidin, or steptavidin). Of course the separation or assembly of reagents in same or different container means is dictated by the types of extraction, amplification or hybridization methods, and detection methods used as well as other parameters including stability, need for preservation etc. It will be understood that different permutations of containers and reagents of the above and foregoing are also covered by the present invention. The kit may also include instructions regarding each particular possible diagnosis, prognosis, theranosis or use, by correlating a combined PDIA3 mRNA level over the PDIA3 mRNA level with a particular diagnosis, prognosis, theranosis or use, as well as information on the experimental protocol to be used.

The methods generally disclosed for detection of PCA3 and PSA mRNA in the WO 2006/066965 A are applicable also for the detection of PDIA3 mRNA levels in the samples according to the present invention, especially for detection these levels in prostate tissue biopsy and blood derived samples.

It is evident that any method or use according to the present invention refers to in vitro methods and uses.

The invention is further described by the following examples and the figures, yet without being limited thereto:

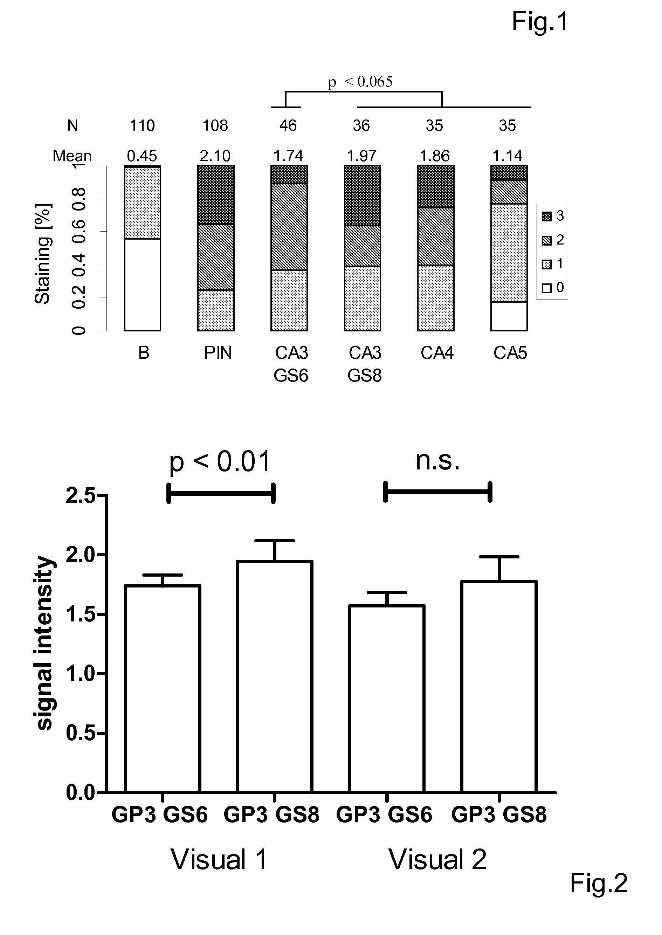

Figure 1 shows the Gleason grade-associated protein expression of PDIA3. (A) Quantification of PDIA3 protein expression in tissue samples via IHC staining intensity was evaluated as no (0), weak (1), intermediate (2) and strong (3) staining. Number of assessed samples (N) and mean staining intensity are indicated for each category. B: Benign tissue; CA3: Gleason Pattern 3; CA4: Gleason Pattern 4; CA5: Gleason Pattern 5; and prostate intraepithelial neoplasia (PIN) tissue;
Figure 2 shows two independent analyses by IHC of PDIA3 expression in GP3 areas of GS6 tumours compared to GP3 areas of GS8 tumours. The results show that the difference is reproducible.

### Examples:

In the present study, a microarray-based gene expression profile analysis of 65 microdissected samples comprising 25 samples of GS 6, 27 samples of GS 8-10 and 13 non cancerous samples was performed. It was sought to identify biological markers of distinct functional groups for the discrimination between low and high grade tumours. Overall, 20 genes were found with a significant alteration in expression in high grade tumours in comparison to low grade tumours. In concordance, two of these genes exhibited Gleason grade associated protein expression in tumour tissues, which can serve as a valuable diagnostic tool.

### Materials & Methods

### Tissue Specimen

Frozen and paraffin-embedded prostate tissue samples were obtained from previously untreated patients who had undergone radical prostatectomy after tumour diagnosis in a PSA based screening program performed in Tyrol by the Department of Urology, Medical University of Innsbruck (Bartsch et al., BJU International 101 (2008), 809-816. The study was approved by the ethics committee at the Medical University of Innsbruck. Immediately after surgery, the prostate specimens were cooled in ice/water and brought to the pathologist who performed a rapid section and isolated a prostate slice that was embedded in Tissue-Tek OCT Compound (Sakura, Tokyo, Japan), snap frozen in liquid nitrogen and stored at -80°C until use. The rest of the prostate was fixed and paraffin-embedded according to standard procedures.

### Tissue Microdissection

For isolation of total RNA, frozen sections were stained with hematoxylin and eosin for pathological analysis and exact localization of the tumours. Parallel unstained slides were used for microdissection. These were pre-treated for 1 min in each of the following pre-cooled solutions: 75% ethanol, RNase-free water, 100% ethanol (twice) and xylene (twice), and air dried. Laser-capture microdissection was performed on a Pix Cell II microdissection microscope (Arcturus, Sunnyvale, CA, USA) using 2,000-5,000 laser impulses for each sample. Tumour samples were isolated from a cohort of Gleason score 6 tumours (Gleason Pattern 3) and a group of Gleason score 8 - 10 tumours (Gleason Patterns 4 and 5). Benign epithelial cell samples were obtained apart from tumour foci from histopathologically normal regions of the same specimens. After microdissection, total RNA was isolated using the PicoPure isolation kit (Arcturus) according to the protocol of the supplier. Quality control was done employing the Agilent Bioanalyzer and picochips.

### Microarray analysis

10ng of RNA isolated from laser-capture microdissected epithelial prostate tumour cells of tissues from patients who had undergone radical prostatectomy were subjected to a two-round amplification using the MessageAmpTM II aRNA Amplification Kit (Applied Biosystems/Ambion, Austin, USA). The quality of amplified RNA (aRNA) fragments was assessed by microcapillary electrophoresis using the Agilent Bioanalyzer 2100 system. Two micrograms of aRNA were subjected to microarray hybridisation as described in (Boer et al., Genome Res. 11 (2001), 1861-1870. Briefly, aRNA was reverse transcribed using SuperScript II reverse transcriptase (Invitrogen, San Diego, CA, USA) and labeled with Cy5-dUTP. Each sample was compared to a common reference (Universal Human Reference RNA; Stratagene, La Jolla, CA, USA) labeled with Cy3-dUTP. Hybridizations were done on 37500-clone human cDNA arrays, representing estimated 22,000 transcripts. Data were analyzed using the GenePix 4.0 software (Axon Instruments, Foster City, USA). Low quality measurements were excluded from further analysis. Raw expression values were pre-processed using ArrayMagic (Buness et al., Bioinformatics 21 (2005), 554-556 and thereby normalized using the VSN method (Huber et al., Bioinformatics 18 (2002), Suppl.1, S96-104.

### Data Analysis and Data Mining

Significance Analysis of Microarrays (SAM) was applied to identify genes differentially regulated between normal tissue, tumour tissue GS 6 and GS ≥ 8 (Tusher et al., PNAS 98 (2001), 5116-5121. A two class unpaired approach with 1000 permutations was used. The False Discovery Rate (FDR) was set below 5%. Results from the SAM analysis were imported into FatiGO (Al-Shahrour et al., Bioinformatics 20 (2004), 578-580 and Ingenuity Pathways Analysis (IPA) software (Ingenuity Systems, Redwood City, CA, USA) to identify genes ontologies that were significantly over- or under-represented.

### Quantitative real-time RT-PCR validation

Verification of expression of selected genes was performed via quantitative real-time RT-PCR using the LightCycler System (Applied Biosystems, Foster City, CA, USA) and the Universal Probe Library System of Roche (Basel, Switzerland). Analyses were performed by using the SDS-software of Applied Biosystems. The expression level of the housekeeping gene β-2-microglobulin was used for normalization, calculated with the 2^{-ΔΔCt} method (Livak et al., Method. Methods 25 (2001), 402-408. Gene expression differences between the tumour Gleason score 6 and Gleason score 8-10 were analyzed using t-test and paired t-test respectively.

### Immunohistochemical Analysis

For validation of expression at the protein level, immunohistochemistry on corresponding paraffin-embedded tissue specimens from the same patient cohort was used. Immunohistochemistry was performed with 5 µm paraffin tissue sections employing the Ventana Discovery - XT staining automate (Roche). Standard CC1 pre-treatment and antigen retrieval was followed by incubation with antibody solution for 1 hr, choice of amplification kit, universal antibody solution for 60 min, staining with DAP map kit and counter stain for 4 min with haematoxylin II bluing reagent (all from Ventana/Roche). For expression analysis of PDIA3 protein the mouse monoclonal antibody MaPERp571 (Abcam) is used at a dilution of 1:10. Specificity of staining was controlled by including a control antibody (DAKO Cytomation, Glostrup, Denmark). Immunoreactivity was then scored by a uropathologist and stratified according to the histology and the Gleason Pattern of the specimens using a 4 point scaling system: 0, no staining, 1, weak staining, 2, intermediate staining, 3, strong staining.

### Apoptosis assay

Human prostate cancer cell lines PC3 and LNCaP were purchased from ATCC (Manassas, VA, USA) and cultured in RPMI 1640 or HAMs F12 medium, respectively. The medium was supplemented with 50 units/ml penicillin, 50 µg/ml streptomycin sulfate, 1% nonessential amino acids, and 10% FBS (all from GIBCO/BRL, Gaithersburg, MD, USA).

PDIA3 siRNA pool [LU-003674-00; siRNA #1; J-003674-09, target sequence: GGAAUAGUCCCAUUAGCAA; siRNA #2; J-003674-10, target sequence: GGGCAAGGACUUACUUAUU; siRNA #3; J-003674-11, target sequence: AGACCCAAAUAUCGUCAUA; siRNA #4; J-003674-12, target sequence: GAGGAGUUCUCGCGUGAUG] was purchased from Dharmacon (Lafayette, CO, USA) and evaluated against a scrambled siRNA control (Qiagen, Hilden, Germany). The siRNA knockdown experiments were performed by plating 0.8 x 10⁴ cells (PC3) and 1 x 10⁴ cells (LNCaP) in a 96-well plate (NUNC, Roskilde, Denmark) overnight. For transfection, siRNA and Lipofectamine 2000 (Invitrogen, Carlsbad, CA, USA) were diluted separately and incubated for 5 min at room temperature. The two solutions were mixed and incubated for 20 min at room temperature. siRNA-Lipofectamine 2000 mixture was then added to the cells, and the plate was mixed by gentle rocking. Transfected cells were incubated at 37°C and 5% CO₂ for 48 h. Knockdown efficiency was verified by qRT PCR.

Induction of apoptosis was performed by adding indicated amounts of Staurosporine (Roche, Mannheim, Germany), Fenretinide (Sigma, Munich, Germany) or Tapsigargin (Sigma) for 6 and 24 hour, respectively. Control cells were left untreated. Activation of apoptosis was determinded by measuring caspase 3 and 7 activities using Caspase-Glo 3/7^{®} Assay (Promega, Madison, WI, USA) following the manufacturer's protocol.

### Results

### mRNA expression analysis revealed large expression differences between GS 6 and GS 8-10 tumours.

To reveal distinct gene expression signatures associated with histological tissue patterns, laser-capture microdissection was applied to selectively isolate pure population of prostate epithelial cancer cells with different Gleason Scores. The expression levels of genes were monitored by hybridization of twice-amplified RNA to cDNA microarrays representing ~ 37500 mapped genes. In total, 65 RNA samples derived from 13 benign and 52 prostate cancer tissue comprising 25 samples with Gleason Score (GS) 6 and 27 samples with GS 8-10 were hybridized. After quality assessment of microarray hybridizations, gene expression profiles were subjected to SAM (Tusher et al., 2001).

For the identification of grade-discriminating genes the expression levels of GS 6 with GS 8-10 tumours were compared. SAM analysis revealed 1141 up-regulated and 54 down-regulated non redundant genes in advanced tumours (FDR 5%). For validation purposes the present data were compared with an independent study from True and coworkers (True et al., PNAS 103 (2006), 10991-10996). True and coworkers reported 86 genes as becoming deregulated during tumour progression from low to high grade. Of these, 17 genes (23%) were identified which all displayed the same tendency.

To identify biological processes associated with tumour progression, gene ontology analysis with genes differentially deregulated between low- and high-grade tumours was performed. In order to extract highly significant canonical pathways, each gene symbol was mapped to its corresponding gene object in the IPA Knowledge Base, and networks were generated algorithmically. Significant canonical pathways were related to actin-mediated processes, e.g. regulation of actin-based motility mediated by Rho-family GTPases (11 of 92 annotated genes, p=5.23E⁻⁰³ ) and actin cytoskeleton signaling (19 of 221 annotated genes, p=1.4E⁻⁰²). In addition, various metabolic processes, including oxidative phosphorylation (22 of 158 annotated genes; p=8.95E⁻⁰⁶) and protein ubiquitination (19 of 205 annotated genes; p= 5.6E⁻⁰³) were deregulated in high-grade tumours. In order to extract as many genes as possible involved in apoptotic processes two further GO analysis tools -FatiGO (Al-Shahrour et al., 2004) and GOstat (Beissbarth et al., Bioinformatics 20 (2004), 1464-1465) were used and a set of 46 genes associated with apoptosis (GOstat p=3.33E⁻⁰⁴) was identified.

Comparisons of gene signatures between normal and tumour tissue lead to the identification of > 2500 deregulated genes (FDR 5%) of which 2390 genes were down and 243 up-regulated. GO analysis performed with this set of genes using IPA Knowledge Base extracts the fructose and mannose metabolism pathway as the one significantly deregulated pathway (2 of 139 annotated genes; p= 4.6E⁻⁰²). In addition, separate SAM analyses between normal and GS 6 or GS8-10 was performed. These analyses revealed 2016 and 2001 down-regulated as well as 463 and 454 up-regulated non redundant genes between normal and GS 6, and normal and GS 8-10 comparisons, respectively. Of these, 1197 genes were deregulated with the same tendency in both tumour groups. Interestingly, three genes (VCAN, CLK1 and TMEM16G) revealed opposite expression levels in these comparisons. VCAN and CLK1 were found to be significantly down-regulated in GS 6, but up-regulated in higher grade tumours in comparison to normal tissue. In agreement with these results, over-expression of VCAN and CLK1 has been described recently between primary prostate cancer and metastatic cancer (Dhanasekaran et al., 2001). In contrast TMEM16G, for which the microarray findings are supported by qRT PCR data, showed the opposite trend. TMEM16G was validated as up-regulated between normal and GS 6 tissue, but down-regulated between normal and GS 8 as well as between GS 6 and GS 8 tissues. In a recent study, TMEM16G was described as a prostate-specific plasma membrane protein promoting cell-cell contact in the prostate cancer cell line LNCaP (Das et al., Cancer Res. 67 (2007), 1594-1601.

### Validation of selected genes by qRT-PCR

To validate the microarray results, 71 genes were selected on the basis of the gene ontology and pathway analyses for confirmation with 57 prostate samples via quantitative real time PCR. B2M (β2-microglobulin) was used as a housekeeping gene due to its even expression in all analyzed patient groups. Focus was laid on genes representing functional groups involved in tumourigenesis like apoptosis, cell morphology, metabolism and ubiquitination. The microarray data of 23 genes were verified as significantly deregulated in the qRT-PCR (Wilcoxon p < 0.1) between low and high-grade tumours. The tendency of 20 of these genes (87%) were in concordance with the microarray results. 36 genes were determined as non-significant and 12 genes exhibited Ct values above detection level.

Deregulation of the apoptotic process plays a major role in tumourigenesis and influences therapeutic outcome. Therefore it was sought to verify deregulated genes involved in apoptotic processes via qRT PCR. Using this method on the same set of samples 11 genes were verified, PDIA3, MAP3K5, ANXA5, VDAC1, NGFRAP1, TEGT, NPM1, VCP, TRAF4, HMGB1, and ROCK1, especially PDIA3, NPM1 and VDAC1 as a preferred triple marker combination, especially for distinguishing between GP3 and GP4 in tumours.

### Immunohistochemistry demonstrates Gleason-grade associated protein expression of PDIA3.

To confirm the present data at the protein level,immunohistochemical analysis of the protein PDIA3 representing the largest functional group (apoptosis) of validated genes was performed. Expression levels were scored according to a 4 point scoring system. Lowest expression levels of PDIA3 protein were seen in benign epithelial cells (Figure 1).

PDIA3 showed a grade-associated protein expression (p < 0.01, Wilcoxon signed rank test) (Figure 1). Interestingly, expression level of PDIA3 in Gleason Pattern 3 (CA3) seems to depend on the accompanied Gleason Pattern. PDIA3 expression was higher in presence of higher Gleason Pattern (CA5) tumours (mean 1.97) than in presence of lower Gleason Pattern (CA3) tumours (mean 1.74). Immunostainings showed cytoplasmic and perinuclear localization of PDIA3.

Table 1 shows the statistical PDIA3 intensities of 0 to 3 (3 being the most intensive staining) of Gleason Pattern 3 region. The table shows the number of counted intensity steps.

| | | **0** | **1** | **2** | **3** | p value (X-square) | X-square |
|---|---|---|---|---|---|---|---|
| **visual 1** | **GS6** | 0 | 17 | 24 | 5 | 0.0082 | 9.6 |
| | **GS8** | 0 | 14 | 9 | 13 | | |
| **visual 2** | **GS6** | 0 | 18 | 24 | 6 | 0.35 | 2.35 |
| | **GS8** | 0 | 8 | 7 | 5 | | |

The results show that PDIA 3 is expressed in Gleason Pattern 3 in dependence of the GS of the surrounding tissue. On the basis of the results of the pathologic analysis a statistically higher expression of PDIA 3 is present in the presence of Gleason Pattern 5 (GS8) in comparison to a Gleason Pattern 3 limited tumour (GS6) (see table 1, visual; Figure 2). In advanced tumours PDIA3 displayed a heterogeneous staining pattern and the variances of intensity distributions were higher.

### Decreased apoptotic activity upon knockdown of PDIA3 in prostate cancer cell lines

PDIA3 was found to be proteins associated with apoptotic processes via pathway analysis. The role for PDIA3 in apoptosis has been largely unexplored. Therefore siRNA-based knockdown of PDIA3 was performed in the human prostate cancer cell lines PC3 and LNCaP. Induction of apoptosis was performed by three different stimuli: Staurosporine (STS), Fenretinide (FenR), Tapsigargin (TG), which are known to activate apoptosis via distinct mechanisms. Each stimulus activated the apoptotic pathway reflected by activation of caspase 3 and/or caspase 7. PDIA3 siRNA treatment revealed a significant decrease of caspase activation in PC3 cells with all stimuli in comparison to control siRNA treated cells. In LNCaP cells similar results were obtained, but were only significant after STS induction. These results indicate a novel, pro-apoptotic role for PDIA3 in prostate cancer cells.

### Antibody staining with PDIA3, NPM1 and VDAC1 antibodies

Antibodies against PDIA3, NPM1 and VDAC1 are all able to distinguish between tumour and benign areas. For differentiation Gleason Pattern 3 and 4 NPM1 was the best marker.

**Table 2: Staining intensities (mean value) of selectd proteins in immunohistochemistry**

| | **PDIA3** | | **NPM1** | | **VDAC1** | |
|---|---|---|---|---|---|---|
| | N | Mean score | N | Mean score | N | Mean score |
| Benign | 55 | 0.5 | 31 | 1.15 | 31 | 0.76 |
| GP 3 | 41 | 1.8 | 26 | 1.77 | 28 | 1.49 |
| GP 4 | 19 | 1.8 | 11 | 2.23 | 13 | 1.8 |
| GP 5 | 18 | 1.3 | 13 | 1.92 | 14 | 1.5 |
| GP 4&5 | 37 | 1.5 | 24 | 2.06 | 27 | 1.65 |
| PIN | 56 | 2.1 | 29 | 2.29 | 30 | 2.11 |
| Inflammation | 10 | 1.2 | 13 | 2.27 | 13 | 1.62 |
| p (Benign vs. GP3) | | <0,0001 | | <0,0001 | | <0,0001 |
| p (GP3 vs. GP4) | | >0,9 | | 0.047 | | 0.17 |

| | | | | | | |
|---|---|---|---|---|---|---|
| N = number of tissue/tumour areas analysed Mean score = mean of lowest and highest expression level within an analysed area Differences of Gleason score 6 and higher Gleason score tumours in their Gleason Pattern 3 areas are low. Here, PDIA3 is the best marker. | | | | | | |

**Table 3: Prediction of higher Gleason score due to staining of Gleason Pattern 3**

| | | **PDIA3** | **NPM1** | **VDAC1** |
|---|---|---|---|---|
| GP3 of GS6 | | 1.75 | 1.83 | 1.53 |
| GP3 of GS7+ | | 1.97 | 1.68 | 1.42 |
| Difference | | 0.22 | -0.15 | -0.11 |

In a multivariant analysis (Table C), NPM1 is also the best marker for discrimination Gleason Pattern 3 and 4; however, the combination of PDIA3, NPM1 and VDAC1 leads to significant results (classification by Random Forest Algorithm; Table D)

**Table 4: Classification of markers and marker combinations:**

| | sensitivity | specifity |
|---|---|---|
| PDIA3 | 0.29 | 0.77 |
| NPM1 | 0.35 | 0.87 |
| VDAC1 | 0.29 | 0.61 |
| PDIA3+NPM1+VDAC1 | 0.41 | 0.71 |

**Table 5: Marker combinations (score sum of PDIA3, NPM1 and VDAC1; score sum is the sum of the single scores; significance is with score sum)**

| **PDIA3+NPM1+VDAC1** | | | |
|---|---|---|---|
| | N | | Mean score sum |
| Benign | 30 | | 2.4 |
| GP 3 | 20 | | 4.9 |
| GP 4 | 10 | | 5.8 |
| GP 5 | 8 | | 4.9 |
| GP 4&5 | 18 | | 5.4 |
| PIN | 26 | | 6.6 |
| p (Benign vs CA3) | | < 0.0001 | |
| p (Benign vs CA4) | | < 0.0001 | |
| p (Benign vs CA5) | | < 0.0001 | |
| p (GP3 mean vs. GP4 mean) | | > 0.034 | |
| p (GP3 mean vs. GP5 mean) | | > 0.6 | |

These results clearly show that the marker combination of PDIA3, NPM1 and VDAC1 are superior to established prostate cancer diagnosis. It is therefore possible to distinguish between Gleason Pattern 3 and 4 in a statistically significant manner.

Recent studies showed that it is important to include different tumour stages of prostate cancer in gene expression analyses to be able to find new diagnostic and prognostic markers. Here, gene expression profiles of low-grade (GS 6) and high-grade prostate cancer (GS 8-10) tissues were generated. In contrast to many other published studies, tumour samples were carefully microdissected down to the single-cell level before RNA isolation. The comparison of these profiles revealed that both tumour subgroups differ by a large number of genes, most of which are up-regulated in high GS tumours. A comparison with another published data set (True et al., 2006) shows that these results are unlikely to be an artifact caused by biases in the microarray analysis, but rather reflect a general trend in transcriptional activation in advanced prostate tumours. Among the detected genes many are involved in pathways which are known to be altered in tumour progression such as apoptosis, morphologic changes, metabolism, and ubiquitin-mediated protein degradation. 20 representatives of these processes were verified via qRT-PCR, and a set of genes discriminating between less and more aggressive tumour forms was identified.

One of the hallmarks of aggressive cancer is the imbalance between cell survival and apoptosis. The present gene ontology analysis revealed that a pronounced number of apoptotic-related genes exhibited expression changes between low and high grade prostate tumours. Thus, for validation, the present analysis was focused on up-regulated anti-apoptotic genes and key players of apoptotic signaling and verified the expression level of selected genes. These data were supported by the analysis of protein expression levels for PDIA3, NPM1 and VDAC1 by IHC.

PDIA3 is a member of the endoplasmatic reticulum stress signalling pathway, and its expression level increases in response to cellular stress. Recently published data demonstrated an anti-apoptotic effect of PDIA3 in the melanoma cell line A375 after induction of ER stress. In contrast, the present study shows a decrease of caspase activity due to reduction of PDIA3 in prostate cancer cell lines. This result shows that PDIA3 functions as a pro-apoptotic protein in the prostate. Therefore over-expression in high grade tumours caused for example by increased stress supports cell death. Taking the present IHC data of PDIA3 into account, PDIA3 protein concentration decreases significantly in CA5 compared to CA4 tissues. In conclusion, a lack of PDIA3 might enhance malignancy in late tumourigenesis. Expression data comparing localized with metastatic prostate cancer showed a down-regulation of PDIA3 (Dhanasekaran et al., 2001). These findings support the mechanism of decreasing expression level of PDIA3 in the late onset of prostate cancer progression. A lack of PDIA3 expression also correlated with increased tumour invasion and advanced stage of gastric cancer and has therefore been proposed as negative prognostic marker. In addition to its function in the ER stress pathway, PDIA3 has garnered recent attention due to its function as a component of the peptide-loading complex of the major histocompatibility complex (MHC) class I pathway. Impairment of this complex in PDIA3 deficient cells would negatively impact the cell surface expression of antigenic peptides, which may help tumours to escape from immune surveillance by cytotoxic T cells. In addition, defective MHC class I antigen surface expression promotes cellular survival through elevated ER stress in a p53 dependent manner. One cause for enhanced survival could be the decrease of apoptosis. However, the present observation of decreased apoptosis after PDIA3 reduction seems unlikely to be mediated via p53 because PC3 is a p53 deficient cell line. Therefore PDIA3 seems to influence apoptotic processes in a p53 independent way. Results of the present IHC data show the high potential for PDIA3 as a diagnostic marker: PDIA3 expression of Gleason Pattern 3 tumours is significantly higher in presence of a Gleason Pattern 5 tumour than in presence of another Gleason Pattern 3 tumour. Therefore expression of PDIA3 depends on environmental factors. Additionally, PDIA3 have been found to be significantly (FDR 5%) up-regulated in tumours harbouring a TMPRSS2 - fusion protein. This underlines the high potential for PDIA3 as discriminating biomarkers in respect of histological grading system and gene arrangement classification.

In conclusion, this study identified a set of genes which discriminates between low and high grade prostate tumours. These genes comprise important functional groups well known to be involved in tumour progression such as apoptosis, morphological changes, metabolism and others. In addition, a grade-associated protein expression of PDIA3, NPM1 and VDAC1 was shown. In particular, PDIA3 protein detection in Gleason Pattern 3 can designate the presence of higher grade tumours in the same tissue. Using the markers identified in addition to recently described markers like DAD1 and MAOA aids in a more patient specific diagnosis. Moreover, the combination of PDIA3, NPM1 and VDAC1 has shown to be specifically suitable for confirming GP3 status of a tissue sample and distinguishing it e.g. from GP4.

## Claims

1. Use of Protein disulfide-isomerase A3 (PDIA3) for distinguishing a significant high-grade prostate carcinoma from insignificant low-grade prostate carcinoma in a tissue sample, especially in a prostate biopsy tissue sample, preferably by the following steps:
- identifying a Gleason 3 region in the tissue sample;
- determining the expression of PDIA3 in the Gleason 3 region identified; and
- identifying the tissue sample as being derived from a significant high-grade prostate carcinoma if the expression level of PDIA3 determined is increased compared to the expression level of PDIA3 in a Gleason 3 region of an insignificant low-grade prostate carcinoma.

2. Use of PDIA3 as a marker for diagnosis of prostate cancer.

3. Use according to claim 1 or 2, **characterised in that** expression of PDIA3, especially in a Gleason 3 region, is determined by immunohistochemical (IHC) methods, by immunofluorescence (IF) methods, by RNA in-situ hybridisation, by reverse transcriptase polymerase chain reaction (RT-PCR), especially quantitative real time RT-PCR (qRT-PCR), or by a combination of these methods.

4. Method for diagnosing prostate cancer in a patient comprising the following steps:
- providing a tissue sample or a body fluid sample of said patient;
- determining the amount of PDIA3 or the amount of expression of PDIA3 in the sample; and
- comparing the amount of PDIA3 or the amount of expression of PDIA3 in the sample with a sample of known prostate cancer status.

5. Method according to claim 4, **characterised in that** the sample is a prostate tissue sample, a sample derived from blood, a saliva sample, a bladder washing sample, a semen sample or a urine sample, especially a prostate biopsy or a plasma or serum sample.

6. Method for distinguishing significant high-grade prostate carcinoma from insignificant low-grade prostate carcinoma in a patient comprising the following steps:
- providing a prostate tissue sample of said patient;
- identifying a Gleason 3 region in the tissue sample;
- determining the expression of PDIA3 in the Gleason 3 region identified; and
- identifying the tissue sample as being derived from a significant high-grade prostate carcinoma if the expression levels of PDIA3 determined are increased compared to the expression levels of PDIA3 in a Gleason 3 region of an insignificant low-grade prostate carcinoma.

7. Use of expression levels of PDIA3 in a Gleason 3 region of a tissue sample of a prostate carcinoma for distinguishing significant high-grade prostate carcinoma from insignificant low-grade prostate carcinoma wherein increased expression levels of PDIA3 identify a significant high-grade prostate carcinoma and wherein non-increased or decreased expression levels of PDIA3 identify an insignificant low-grade prostate carcinoma.

8. Use of a kit for detecting the amount of PDIA3 in a sample, comprising detection agents for PDIA3 for diagnosing a tissue sample or a sample of a body fluid for prostate cancer, especially for distinguishing significant high-grade prostate carcinoma from insignificant low-grade prostate carcinoma.

9. Use of a kit for detecting the expression level of PDIA3 in a sample, comprising detection agents for PDIA3 mRNA for diagnosing a tissue sample or a sample of a body fluid for prostate cancer, especially for distinguishing significant high-grade prostate carcinoma from insignificant low-grade prostate carcinoma.
